# EUROPEAN PATENT SPECIFICATION

(11) **EP 2 376 880 B1**
(45) Date of publication and mention of the grant of the patent: **12.02.2020**
(21) Application number: 09833742.1
(22) Date of filing: 17.12.2009
(51) Int. Cl.: G01J 3/32, G01J 3/44, G01N 21/65

(54) **METHOD AND SYSTEM FOR DETECTING SMALL AMOUNTS OF SUBSTANCES**
VERFAHREN UND SYSTEM ZUM DETEKTIEREN KLEINER MENGEN VON SUBSTANZEN
MÉTHODE ET SYSTÈME DE DÉTECTION DE PETITES QUANTITÉS DE SUBSTANCES

(30) Priority: 18.12.2008 SE 0802612
(43) Date of publication of application: 19.10.2011
(73) Proprietor: Totalförsvarets Forskningsinstitut FOI, 164 90 Stockholm (SE)
(72) Inventor: Henric, Östmark, S-141 41 Huddinge (SE)
(74) Representative: Hedefält, Dag
(86) International application number: PCT/SE2009/051440
(87) International publication number: WO 2010/071579

(56) References cited:
- EP-A1- 1 933 127
- WO-A2-2006/013021
- US-A- 6 040 906
- US-A1- 2007 081 156
- US-A1- 2007 222 981
- US-A1- 2008 198 365
- US-B1- 7 333 190
- US-B1- 7 333 190

## Description

### TECHNICAL FIELD

The present invention relates to detection of substances or molecules, in particular detection of very small amounts of substances or of particles of substances at stand-off distances.

### BACKGROUND

A substance, e.g. an explosive substance or a controlled drug, that is hit by a laser beam will reflect or scatter most of the received light. The reflected and scattered light will mainly have the same wavelength as the received light, however some of the scattered light will be wavelength-shifted, this being "Raman scattered" light. The distribution of this scattered light comprising light of a plurality of wavelengths is called a "Raman spectrum".

The Raman spectrum for each substance or more precisely for each type of molecule thus, in the way similar to other spectra, comprises one or more wavelength bands/lines and is molecule-specific. Every band or line in the Raman spectrum corresponds to a vibrational mode in the molecule. Due to the unique Raman spectrum for each molecule a substance can be identified by comparing a measured Raman spectrum to reference spectra.

For stand-off detection, i.e. remote detection or detection at some distance, a laser beam is directed at the substance and the reflected and scattered light is collected using a telescope. The Raman spectrum is measured using an optical filter system and an optical sensor. In order to identify the substance or molecule for which the Raman spectrum has been measured a signal processing algorithm is used to compare the measured spectrum to various spectra stored in a database. Remote detection can be assumed to be made at distances, these distances called stand-off distances, larger than those ordinarily in a laboratory where the substance to be examined is placed in a direct vicinity of a spectrometer, the distance generally being taken as larger than 50 cm or commonly larger than 5 m and often in the range of 10 -100 m.

A difficult task in substance detection is to identify small amounts, also called trace amounts, of a relevant substance or a substance searched for that is located amid many other substances. In the detection, conventionally a signal from the relevant substance is obtained together with signals from the other substances, where the latter signals can be called noise. The lower the proportion of signal to noise, this called the signal-to-noise ratio (SNR), is, the more difficult the detection becomes. This problem is common to a multitude of applications for particle detection.

One such task is to identify substances present in fingerprints, where a number of other substances may also be present. Substances in fingerprints usually present themselves in the form of particles. Therefore, in order to maximize the SNR it is desirable to perform the detection using the area of the fingerprint where the ratio of relevant particles to irrelevant particles is as high as possible. The area having the largest SNR is obviously the area of one relevant particle, excluding all surrounding area. This is true not only for fingerprints, but for every application where it is desirable to detect trace amounts of substances in a cluttered environment.

To achieve this, two objectives have to be met. First, the detector used must have a near part-ide-size resolution at stand-off distances and second, the detector has to be capable of locating near particle-sized objects at stand-off distances.

In the examination of the present patent application US 2007/0081156A1 was cited. While it shows some of the characteristics of the present invention, it does not show stand-off distances of 10-100 meters, which are used in the present invention. Furthermore, it is directed towards detecting bioagents, not hazardous molecules of a definite kind. The mere mentioning in passing of chemical warfare agents detection or hazardous material monitoring does not give information enough about such a use.

### SUMMARY

It is an object of the present invention to provide a method according to claim 1 and a device according to claim 6 for detecting very small amounts of substances.

Generally, an area of detection or of interest can be divided into a plurality of smaller areas, herein called subareas, where the size of each subarea is comparable to the size of one or of a plurality of, e.g. a few such as 2 - 5 or better 2 - 3, particles. Since such a subarea will contain only one or only a few particles the SNR thereof will be high if any relevant particle is present. In extreme cases, such as for detecting special organic molecules, the subareas may even have a size comparable to the of a single or of a few molecules.

By grouping a plurality of subareas to form a larger area and simultaneously detecting all individual subareas included in said plurality, any subarea of the larger area having one or a few relevant particles can be found.

Generally, in detecting a substance, a laser beam is directed at a target, focusing a telescope at a place on the target illuminated by the laser beam and depicting the telescope image on a camera having several picture elements. Each picture element will then correspond to an individual element subarea on the target. An optical filter inserted between the telescope and the camera provides spectral information regarding the substances on the target. By repetitiously changing the band-pass wavelength of the optical filter, and collecting the response of the camera for each filter setting, the individual spectral response for each element subarea can be determined.

The present invention is defined in the appended claims.

### BRIEF DESCRIPTION OF THE DRAWINGS

The invention will be better appreciated from a consideration of the following detailed description of non-limiting embodiments presented hereinbelow with reference to the accompanying drawings, in which:
- Fig. 1 is a schematic of a detection system for detecting small amounts of substances,
- Fig. 2 is a schematic illustrating how particles are imaged onto individual pixels,
- Fig. 3 is a schematic similar to Fig. 1 having also a wavelength separating device, and
- Fig. 4 is a schematic similar to Fig. 2 for an illuminated strip imaged onto corresponding pixels.

### DETAILED DESCRIPTION

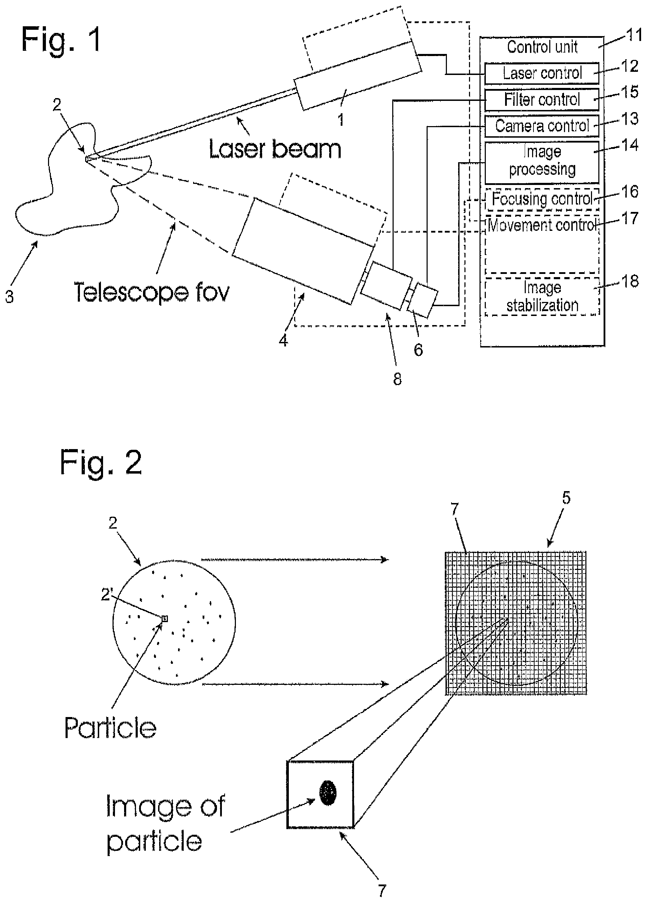

Fig. 1 is a schematic of apparatus for standoff Raman spectroscopy, i.e. Raman spectroscopy for an object located at a relatively large distance of the device for collecting Raman scattered light. A monochromatic light beam is issued by a laser 1 and is directed at an area 2 or region of a target 3. When the light of the light beam hits the area, most of the light is scattered. Also, most of the scattered light has the same wavelength as the light hitting the area. However, a small fraction of the scattered light has shifted wavelengths. The scattered light is collected by an optical telescope 4 that is set to sharply depict the area 2 illuminated by the laser beam. The optical telescope obviously collects all light, including both reflected and scattered light, issued from the depicted area in the direction of the telescope, the reflected light constituting noise. The telescope image is depicted on the light sensitive surface 5 of a camera 6, the light sensitive surface comprising a plurality of picture elements 7, also called pixels, see Fig. 2. Thus, the light sensitive surface may be called a pixel array. Each of the picture elements then corresponds to an individual element subarea 2' included in the illuminated area 2 of the target 3.

Some device for obtaining spectral information about the substances in the illuminated area 3 of the target such as an optical filter 8 can be inserted between the telescope 4 and the camera 6. The optical filter can be the band-pass type, i.e. it can be designed to allow transmission of light only within a wavelength window. By repetitiously changing the setting of the optical filter, such as moving the wavelength window of the optical filter 8, and collecting the response of the camera 6 for each chosen setting or wavelength window, the individual spectral response for each of the element subareas 2' included in the illuminated area 2 can be determined.

As can be seen in Fig. 2 the size of the image of a particle may e.g. roughly be of the same magnitude as a pixel 7 and then the particle may also have a size approximately corresponding to that of the element subarea 2' that corresponds to the pixel. The pixel size and the stand-off distance determine the design parameters of the optical system. The magnification of the optical system may be determined so that the element subarea corresponding to a pixel has a size dependent on characteristics of the relevant substances and their particle size distribution, and considering the background noise, as discussed above, i.e. the contribution from irrelevant substances in the signal resulting from detection in the light sensitive surface 5 of the camera 6, the physical detection method used and the desired SNR. The particle size is typically in the range of 1 - 100 µm and stand-off distances can e.g. be up to 1000 m.

The shape of the pixels 7 and the shape of the pixel array 5 may be arbitrary. The rectangular or square picture element and the rectangular pixel array seen in Fig, 2 represent the typical shapes found in commercial cameras, however, a single row or a single column of pixels or a non-regular or non-contiguous configuration of pixels may also be used.

The detection method used can be ordinary Raman spectroscopy. Other detection methods that can be used include variations of Raman Spectroscopy, e.g. near-resonant Raman spectroscopy and resonance-enhanced Raman spectroscopy (RRS or RERS), and similar methods such as Laser-Induced Fluorescence (LIF).

The optical filter 8 may e.g. be an LCTF (Liquid Crystal Tunable Filter) or an AOTF (Acousto-Optical Tunable Filter). The laser 1 can be an Nd-YAG pulsed laser but other lasers such as excimer lasers, e.g. KrF lasers, may also be used. Some applications may also use continuous-wave (CW) lasers. Some applications may require tunable lasers for e.g. fluorescence rejection and resonance enhancement.

The relevant substances suitable to detect can include, but are not limited to explosives, explosives markers and explosives precursors. Other substances of interest may be TICs (Toxic Industrial Chemicals), CWAs (Chemical Warfare Agents), drugs, drug-pre-cursors and hazardous substances in general. Also, non-hazardous substances indicative of a hazardous activity and/or of hazardous contents may be of interest.

The apparatus can include a control unit 11 comprising units or modules for executing various tasks. Thus, a laser control module 12 can be connected to the laser 1 for controlling when light pulses are emitted and the length of the pulses. A camera module 13 can be connected to the camera 6 for controlling when the pixels 7 of the light sensitive surface 5 are open for detecting light and for controlling the length of their detection time periods. The signals from the camera can be input to an image processing unit 14 which e.g. can select the pixels, the signals from which are suited for analysis. A filter module 15 is connected to the optical filter 8 for controlling the filtering characteristics thereof.

As appears from the description above, the basic procedure when searching for a specific substance or molecule in the target 3 can be as follows.
1. The laser 1 is set so that its emitted light beam is directed to an area 2 on the target 3.
2. The telescope 4 is set so that it sharply or distinctly depicts the illuminated area 2.
3. The optical filter 8 is set to allow light of a first wavelength band to pass.
4. The camera 6 is activated to take a picture. The signal from each pixel 7 is fed to the control unit 11 and stored.
5. The setting of the optical filter 8 is changed to allow light of another wavelength to pass.
6. The steps 4. and 5. are repeated until signals from the camera 6 for light of each wavelength band of interest have been recorded.
7. The stored signals for a relevant one of the pixels 7 or for each one of a group of pixels are analyzed by e.g. comparing them to already stored signal patterns. The result of the comparison is stored.
8. The direction of the laser beam is changed so that it hits another area 2 of the target 3.
9. The steps 2. - 7. are repeated for this another illuminated area 2.
10. The stored results are evaluated.

In the comparing step 7. the relevant pixel 7 may be a single pixel which corresponds to the total area 2 illuminated by the laser beam, i.e. the areas 2 and 2' may be identical. If the area 2 illuminated by the laser beam is depicted on a group of pixels 7, the signals stored for each pixel in the group can be analyzed individually or the signals from all pixels in the group can be summed for each setting of the optical filter 8 and the resulting sums can then be analyzed.

Generally, the method can be adapted to various special cases by e.g. selecting the location of the illuminated area 2 and the shape thereof and by analyzing the signals obtained for e.g. groups of pixels 7 together with each other. Such a group of pixels then correspond to a subarea on the target 3 included in the illuminated area 2 but larger than each of the element subareas 2'. Special embodiments will now be described.

In the case where the telescope 4 is exposed to mechanical shock and vibration, some stabilization device or method can be used, e.g. mechanical or optical stabilization. Also image processing such as that performed in the image processing module 14 can be used to stabilize the detection. In particular, the laser 1 can be set to emit light pulses of e.g. the length 10 ns. The camera control module 13 controls the camera 6 to be open for detection only within suitably set time windows so that the pixels 7 only detect received light, i.e. taking pictures, during these time windows. The time windows are e.g. set to have a selected delay in relay to each issued light pulse and to have a suitably selected length to give an optimum detection of only light scattered from the illuminated subarea. If the telescope 4 is moving during the detection, the detected signals from each pixel 5 will not come from the same small element subarea 2' of the object 3. Before evaluating the detected signals, an image stabilizing procedure can be used, e.g. executed in an image stabilization module 18, in which the taken pictures are compared to each other and if necessary displaced, so that in all the resulting pictures the signals for each pixel come from the same element subarea 2' of the object 3. Finally, the resulting pictures are analyzed by e.g. summing the signals for each pixel 7 of the selected subarea and each setting of the optical filter 8. These sums then constitute the measured spectrum of scattered light for the selected subarea.

Image processing may be used to identify areas on the target which are of particular interest for detection. First an overview picture of the target 3 for some suitable wavelength band can be captured and thereupon the captured picture is analyzed for images of objects matching already captured pictures of objects in a built-in or externally available database, not shown. Examples of such objects include door handles, which are likely to contain trace amounts from fingerprints. The detector system may then be manually or automatically directed at one or more of such identified areas.

Image processing may also be used to match patterns of detected particles to patterns in a database, not shown. An object of interest may first be analyzed for the existence of particles of substances, the spectra of which are included in the database. Then the location of the particles in a picture of the whole object is matched against known patterns of particle location.

An area and/or an object can be scanned using:
1. A geographically stationary detection system in which the optical devices needed for detection, such as the laser 1, the telescope 4, the optical filter 8 and the camera 6, are mounted to angularly move as one unit, in particular having pan and tilt functions, These functions can e.g. be controlled by a movement control module 17. Also, for setting the focusing of the telescope 4, a focusing control module 16 can be used. By angularly moving the optical devices, i.e. panning and/or tilting them as one unit, an object or a surface can be continuously scanned, capturing overlapping pictures of the object or surface. Image processing is used to produce resulting pictures of the whole object or surface, so that as above each picture element 7 in the resulting pictures corresponds to one, and only one, small element subarea 2' of the object 3 or surface of interest. The resulting pictures can then be analyzed to produce spectra of light scattered from subareas of the object or surface.
2. A geographically moving detection system, e.g. mounted on a vehicle, not shown, so that the optical devices needed for detection are rigidly attached to the vehicle. The optical devices are directed to and continuously scan an object 3 or a surface by virtue of the geographical movement. Image processing is as above used to produce resulting pictures of the whole object or surface, so that as above each picture element 7 in the resulting pictures corresponds to one, and only one, small element subarea of the object or surface. The resulting pictures can then be analyzed to produce spectra of light scattered from subareas of the object or surface.
3. A combination of 1. and 2., i.e. a geographically moving detection system in which the optical devices needed for detection are mounted to have pan and tilt functions.

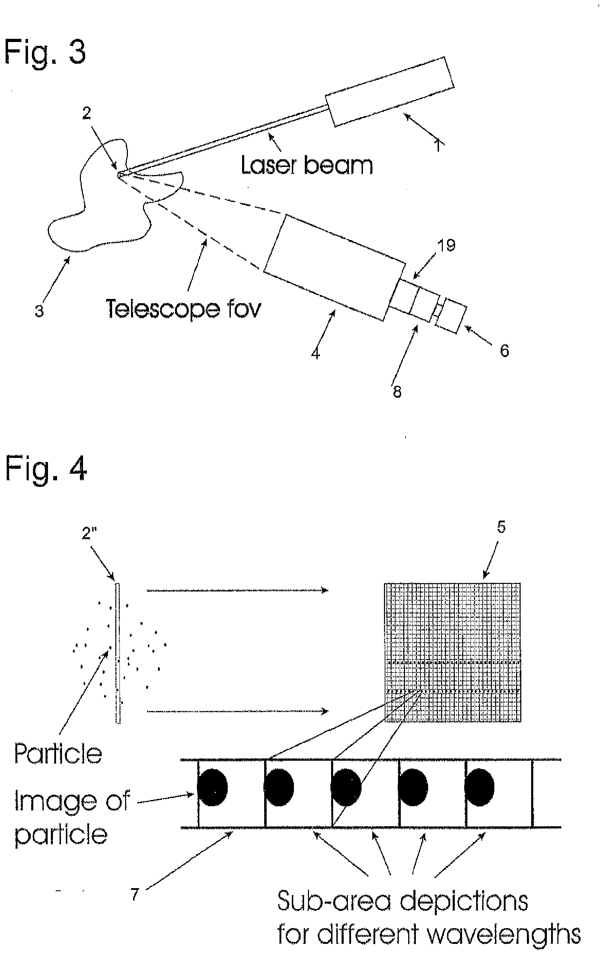

The laser beam may be formed or shaped, e.g. by a suitable optical system, not shown, to have a cross-section having the shape of a thin bar or a thin straight line so that it illuminates a corresponding strip-shaped area 2" of the target 3, i.e. of the object of interest, see Fig. 4. The width of the strip-shaped area can correspond to the width of a subarea of interest. In particular it can correspond to or possibly slightly exceed, such as not exceeding by more than 25 % or better 10 %, the width or height of an area that is depicted on a single pixel, i.e. of an element subarea 2'. The light sensitive surface 5 of the camera 6 may then include only a single linear array of pixels 7, i.e. pixels arranged along a line such as in one row or in one column. The pixels may as in conventional cameras have a rectangular or square shape but they could also be e.g. polygonal. The light from the illuminated strip-shaped area or thin line 2" on the object 3, i.e. both reflected and scattered light from this area, is projected onto the linear pixel array in the camera 6. The illuminated thin line may be oriented horizontally, vertically or in an arbitrary direction.

An area and/or an object can be scanned using a "bar-laser" as described above. The scanning can be made from a stationary geographical location using a detection device having pan and tilt functions as above. The scanning can alternatively be performed in the "moving" way described above. In the latter case, the detection device may optionally also have a pan function or a tilt function or both these functions.

A laser beam formed to have a cross-section of a bar or a thin straight line as described above can also be used together with a conventional rectangular light sensitive surface 5 having a plurality of horizontal rows and a plurality of vertical columns of pixels 7 as seen in Fig. 4. Then the area of the light sensitive surface 5 that corresponds to the illuminated linear area 2" on the target 3 is active for detection. In order to improve the speed of detection, a wavelength dispersive device 19 of an edge or linear type, e.g. a cylinder-segment lens, a prism or a diffraction grating, see Fig. 3, can be used. The wavelength dispersive device can be placed to deflect the collected reflected and scattered light output from the telescope 4 before it passes onto the array 5 of pixels 7. The wavelength dispersive device 19 will for a proper positioning deflect the light so that light of different wavelengths hits different areas of the array 5 of pixels. Then, usually light of the longest wavelengths hits a straight line of pixels at one end or side of the pixel array and light of the shortest wavelengths hits a straight line of pixels at the opposite end or side of the pixel array. Depending on the layout of the pixel array the information or signals obtained from a row of pixels, or a column, then represents/represent the intensity of light, scattered from the corresponding linear area of the target and having wavelengths only within a narrow wavelength band, i.e. the spectral response for a narrow wavelength band from the illuminated area of the target.

For investigating an object 3, a laser beam which illuminates an area 2 of the object 3 corresponding to one or more pixels 7 can be used. The spectrum is obtained in one measurement using spectral-spreading techniques or several measurements of one spectral band at a time. The beam is moved to the next subarea, that can be adjacent or non-adjacent, and another measurement is made. The image of the total object 3 is obtained by adding the responses from the individual subareas for which measurements have been made.

The detection system as described herein can be used in ordinary land-based vehicles but also in un-manned vehicles, e.g. in a UAV (Unmanned Aerial Vehicle) or in a naval vehicle.

It should be understood herein and in the claims hereof that such terms as "top" and "bottom", "upwardly" and "downwordly", "width" and "height" and the like have been used for illustration purposes only, in order to provide a clear and understandable description and claiming of the invention.

While specific embodiments of the invention have been illustrated and described herein, it is realized that numerous other embodiments may be envisaged and that numerous additional advantages, modifications and changes will readily occur to those skilled in the art without departing from the scope of the invention. Therefore, the invention in its broader aspects is not limited to the specific details, representative devices and illustrated examples shown and described herein. Accordingly, various modifications may be made without departing from the scope of the general inventive concept as defined by the appended claims. Numerous other embodiments may be envisaged without departing from the scope of the invention.

## Claims

1. A method of detecting trace amounts of substances in a cluttered environment at stand-off distances for determining whether an area of detection on an object contains a hazardous substance, the area or detection being illuminated with light of a definite wavelength and the scattered light being analyzed in order to obtain a Raman-spectrum or a similar spectrum, the light scattered by the object being collected and concentrated by an optical system, in particular an optical system of the telescope type, depicting the image on a light detection device, **characterized in**
**that** the stand-off distance is ranging from 10 m to 100 m,
**that** the hazardous substance consists of molecules of a definite kind, in particular an explosive or a component of an explosive,
**that** the area of detection is divided into a plurality of smaller areas, called subareas, where the size of each subarea is comparable to the size of one or a few particles of molecules of the definite kind,
**that** a plurality of subareas is grouped to form a larger area and all individual subareas included in said plurality being simultaneously detected by means of the image from the optical system being depicted on the light detection device having several picture elements in a picture element array, wherein each picture element corresponds to an individual subarea, and
**that** in analyzing the scattered light, the detection signals from each of the individual picture elements are individually analyzed.

2. A method according to claim 1, **characterized in that** the collected light is filtered before it is detected so that light of only one wavelength range is analyzed.

3. A method according to claim 2, **characterized in that** the band-pass wavelength of the optical filter is repetitiously changed and the analyzing of the scattered light is executed consecutively for each of a plurality of wavelength ranges.

4. A method according to anyone of claims 1 - 3, **characterized in that** the illuminated area of the surface of the object is a line region or a strip-shaped area, in particular a strip-shaped area having a width corresponding to or slightly exceeding the width or the height of an area that is depicted on a single one of the individual picture element.

5. A method according to claim 4, **characterized in that** the collected light is passed through a wavelength dispersive device of an edge or linear type having an edge or axis oriented so that the light collected from the line region hits parallel lines of the individual picture elements and the light collected from the line region hitting each of these lines includes only wavelengths of a corresponding individual wavelength band.

6. A system of detecting trace amounts of substances in a cluttered environment at stand-off distances for determining whether an area of detection on an object contains a hazardous substance, comprising a light source for illuminating the area of detection with light of a definite wavelength, an optical system, in particular an optical system of the telescope type, for collecting and concentrating light from the illuminated area of the surface of the object, a light detection device receiving the collected and concentrated light, and an analysis unit for obtaining, from signals from the light detection device, a Raman-spectrum or a similar spectrum, **characterized in**
**that** the stand-off distance is ranging from 10 m to 100 m,
**that** the hazardous substance consists of molecules of a definite kind, in particular an explosive or a component of an explosive,
**that** the light detection device comprises a light sensitive surface including a plurality of individual picture elements arranged as a picture element array, each of the individual picture elements providing detection signals, and that the analysis unit is adapted to divide the area of detection into a plurality of smaller areas, called subareas, where the size of each subarea is comparable to the size of one or a few particles of molecules of the definite kind, group a plurality of subareas to form a larger area, and simultaneously detect all individual subareas included in said plurality by the image from the optical system being depicted on the light detection device and analyze individually the detection signals from each of the individual picture elements, wherein each picture element corresponds to an individual subarea.

7. A system according to claim 6, **characterized by** a filter for filtering the collected light before being received by the light detection device, so that light of only one wavelength range is detected.

8. A system according to claim 7, **characterized in that** the filter is tunable so that the light detection device can detect light for each of a plurality of wavelength ranges consecutively by tuning the filter accordingly.

9. A system according to anyone of claims 6-8, **characterized in that** the light source is arranged so that the illuminated area of the surface of the object is a line region or a strip-shaped area, in particular a strip-shaped area having a width corresponding to or slightly exceeding the width or the height of an area that is depicted on a single one of the individual picture element.

10. A system according to claim 9, **characterized by** a wavelength dispersive device of an edge or linear type arranged so that the collected and concentrated light is passed therethrough, the wavelength dispersive device being an edge or linear type having an edge or axis oriented so that the light collected from the line region hits parallel lines of the individual picture elements and the light collected from the line region hitting each of these lines includes only wavelengths of a corresponding individual wavelength band.

11. A system according to claim 10, **characterized in that** the wavelength dispersive device is a cylinder-segment lens, a prism or a diffraction grating.

## Patentansprüche

1. Verfahren zum Detektieren von Spurenmengen von Substanzen in einer unübersichtlichen Umgebung in Abrückabständen zum Bestimmen, ob ein Detektionsbereich auf einem Objekt eine gefährliche Substanz enthält, wobei der Detektionsbereich mit Licht einer definierten Wellenlänge beleuchtet und das gestreute Licht analysiert wird, um ein Raman-Spektrum oder ein ähnliches Spektrum zu erhalten, wobei das von dem Objekt gestreute Licht von einem optischen System, insbesondere einem optischen System vom Typ eines Teleskops, gesammelt und konzentriert wird, das das Bild auf einer Lichtdetektionsvorrichtung darstellt, **dadurch gekennzeichnet,**
**dass** der Abrückabstand von 10 m bis 100 m beträgt,
**dass** die gefährliche Substanz aus Molekülen einer bestimmten Art besteht, insbesondere aus einem Sprengstoff oder einem Bestandteil eines Sprengstoffs,
**dass** der Detektionsbereich in eine Vielzahl von kleineren Bereichen, den sogenannten Teilbereichen, unterteilt ist, wobei die Größe jedes Teilbereichs mit der Größe von einem oder einigen wenigen Teilchen von Molekülen der bestimmten Art vergleichbar ist,
**dass** eine Vielzahl von Teilbereichen gruppiert wird, um einen größeren Bereich zu bilden, und alle in der Vielzahl eingeschlossenen individuellen Teilbereiche gleichzeitig mittels des Bildes aus dem optischen System detektiert werden, das auf der Lichtdetektionsvorrichtung mit mehreren Bildelementen in einem Bildelementfeld dargestellt wird, wobei jedes Bildelement einem individuellen Teilbereich entspricht,
und
**dass** bei der Analyse des gestreuten Lichts die Detektionssignale von jedem der einzelnen Bildelemente einzeln analysiert werden.

2. Verfahren nach Anspruch 1, **dadurch gekennzeichnet, dass** das gesammelte Licht vor dem Detektieren gefiltert wird, so dass Licht mit nur einem Wellenlängenbereich analysiert wird.

3. Verfahren nach Anspruch 2, **dadurch gekennzeichnet, dass** die Bandpasswellenlänge des optischen Filters wiederholt geändert wird und die Analyse des gestreuten Lichts für jeden der mehreren Wellenlängenbereiche nacheinander durchgeführt wird.

4. Verfahren nach einem der Ansprüche 1-3, **dadurch gekennzeichnet, dass** der beleuchtete . Bereich der Oberfläche des Objekts ein Linienbereich oder ein streifenförmiger Bereich ist, insbesondere ein streifenförmiger Bereich mit einer Breite, die der Breite oder Höhe eines Bereichs entspricht oder diese etwas überschreitet, der auf einem einzelnen der einzelnen Bildelemente dargestellt ist.

5. Verfahren nach Anspruch 4, **dadurch gekennzeichnet, dass** das gesammelte Licht durch eine Wellenlängendispersionsvorrichtung eines Kanten- oder Lineartyps mit einer Kante oder Achse geleitet wird, die ausgerichtet ist, so dass das aus dem Linienbereich gesammelte Licht auf parallele Linien der einzelnen Bildelemente trifft und das aus dem Linienbereich gesammelte Licht, das auf jede dieser Linien trifft, nur Wellenlängen eines entsprechenden individuellen Wellenlängenbandes einschließt.

6. System zum Detektieren von Spurenmengen von Substanzen in einer unübersichtlichen Umgebung in Abrückabständen zum Bestimmen, ob ein Detektionsbereich auf einem Objekt eine gefährliche Substanz enthält, umfassend eine Lichtquelle zum Beleuchten des Detektionsbereichs mit Licht einer definierten Wellenlänge, ein optisches System, insbesondere ein optisches System vom Typ eines Teleskops, zum Sammeln und Konzentrieren von Licht aus dem beleuchteten Bereich der Oberfläche des Objekts, eine Lichtdetektionsvorrichtung, die gesammeltes und konzentriertes Licht empfängt, und eine Analyseeinheit zum Erhalten eines Ramanspektrums oder eines ähnlichen Spektrums aus Signalen von der Lichtdetektionsvorrichtung, **dadurch gekennzeichnet, dass** der Abrückabstand von 10 m bis 100 m beträgt,
dass die gefährliche Substanz aus Molekülen bestimmter Art besteht, insbesondere aus einem Sprengstoff oder einem Bestandteil eines Sprengstoffs,
dass die Lichtdetektionsvorrichtung eine lichtempfindliche Oberfläche mit einer Vielzahl von einzelnen Bildelementen umfasst, die als Bildelementfeld angeordnet sind, wobei jedes der einzelnen Bildelemente Detektionssignale bereitstellt, und
dass die Analyseeinheit dazu ausgelegt ist, den Detektionsbereich in eine Vielzahl von kleineren Bereichen, den sogenannten Teilbereichen, aufzuteilen, wobei die Größe jedes Teilbereichs mit der Größe von einem oder einigen wenigen Teilchen von Molekülen der bestimmten Art vergleichbar ist,
eine Vielzahl von Teilbereichen zu gruppieren, um einen größeren Bereich zu bilden, und gleichzeitig alle einzelnen Teilbereiche, die in der Vielzahl eingeschlossen sind, durch das Bild aus dem optischen System, das auf der Lichtdetektionsvorrichtung abgebildet ist, zu detektieren und die Detektionssignale aus jedem der einzelnen Bildelemente einzeln zu analysieren, wobei jedes Bildelement einem einzelnen Teilbereich entspricht.

7. System nach Anspruch 6, **gekennzeichnet durch** einen Filter zum Filtern des gesammelten Lichts, bevor es von der Lichtdetektionsvorrichtung empfangen wird, so dass Licht nur eines Wellenlängenbereichs detektiert wird.

8. System nach Anspruch 7, **dadurch gekennzeichnet, dass** der Filter abstimmbar ist, so dass die Lichtdetektionsvorrichtung Licht für jeden aus einer Vielzahl von Wellenlängenbereichen nacheinander durch entsprechendes Abstimmen des Filters detektieren kann.

9. System nach einem der Ansprüche 6-8, **dadurch gekennzeichnet, dass** die Lichtquelle so angeordnet ist, dass der beleuchtete Bereich der Oberfläche des Objekts ein Linienbereich oder ein streifenförmiger Bereich ist, insbesondere ein streifenförmiger Bereich mit einer Breite, die der Breite oder der Höhe eines Bereichs entspricht oder diese etwas überschreitet, der auf einem einzelnen des jeweiligen Bildelements abgebildet ist.

10. System nach Anspruch 9, **gekennzeichnet durch** eine Wellenlängen-Dispersionsvorrichtung eines Kanten- oder Lineartyps, die so angeordnet ist, dass das gesammelte und konzentrierte Licht **durch** sie hindurch geleitet wird, wobei die Wellenlängen-Dispersionsvorrichtung ein Kanten- oder Lineartyp mit einer Kante oder Achse ist, die so ausgerichtet ist, dass das aus dem Linienbereich gesammelte Licht auf parallele Linien der einzelnen Bildelemente trifft und das aus dem Linienbereich gesammelte Licht, das auf jede dieser Linien trifft, nur Wellenlängen eines entsprechenden individuellen Wellenlängenbandes einschließt.

11. System nach Anspruch 10, **dadurch gekennzeichnet, dass** die Wellenlängendispersionsvorrichtung eine Zylindersegmentlinse, ein Prisma oder ein Beugungsgitter ist.

## Revendications

1. Procédé de détection de quantités infimes de substance dans un environnement à des distances données pour permettre de déterminer si une zone de détection située sur un objet renferme une substance dangereuse, la zone de détection étant éclairée avec de la lumière ayant une longueur d'ondes définie et la lumière dispersée étant analysée de façon à obtenir un spectre Raman ou un spectre similaire, la lumière dispersée par l'objet étant recueillie et concentrée par un système optique, en particulier un système optique de type télescope représentant l'image sur un dispositif de détection de la lumière,
**caractérisé en ce que**
la distance donnée est située dans la plage de 10 m à 100 m,
la substance dangereuse est constituée par des molécules d'un type défini, en particulier un explosif ou un composant d'un explosif,
la zone de détection est subdivisée en un ensemble de zones plus petites dites sous-zones, la dimension de chaque sous-zone étant comparable à la dimension d'une ou d'un petit nombre de particule(s) de molécules du type défini,
un ensemble de sous-zones est regroupé pour former une zone plus grande, et toutes les sous-zones individuelles comprises dans cet ensemble sont détectées simultanément au moyen de l'image du système optique représentée sur le dispositif de détection de la lumière ayant plusieurs pixels dans un réseau de pixels, chaque pixel correspondant à une sous-zone individuelle,
lors de l'analyse de la lumière dispersée, les signaux de détection de chacun des pixels individuels étant analysés individuellement.

2. Procédé conforme à la revendication 1,
**caractérisé en ce que**
la lumière recueillie est filtrée avant d'être détectée de sorte que de la lumière d'une seule plage de longueurs d'onde soit analysée.

3. Procédé conforme à la revendication 2,
**caractérisé en ce que**
la longueur d'onde passe-bande du filtre optique est modifiée de façon répétitive et l'analyse de la lumière dispersée est effectuée successivement pour chacune des plages de longueurs d'onde de l'ensemble de longueurs d'onde.

4. Procédé conforme à l'une quelconque des revendications 1 à 3,
**caractérisé en ce que**
la zone éclairée de la surface de l'objet est une région linéaire ou une zone en forme de bande en particulier une zone en forme de bande ayant une largeur correspondant à ou légèrement supérieure à la largeur ou à la hauteur d'une zone qui est représentée sur un seul des pixels individuels.

5. Procédé conforme à la revendication 4,
**caractérisé en ce que**
la lumière recueillie est transmise au travers d'un dispositif de dispersion de longueurs d'onde à bord ou linéaire ayant un bord ou un axe orienté de sorte que la lumière recueillie provenant de la région linéaire frappe des lignes parallèles des pixels individuels et que la lumière recueillie provenant de la région linéaire frappant chacune de ces lignes ne renferme que des longueurs d'onde d'une bande de longueurs d'onde individuelles correspondante.

6. Système de détection de quantités infimes de substance dans un environnement encombré à des distances données pour permettre de déterminer si une zone de détection située sur un objet renferme une substance dangereuse, comprenant une source de lumière permettant d'éclairer la zone de détection avec de la lumière ayant une longueur d'onde définie, un système optique, en particulier un système optique de type télescope pour permettre de recueillir et de concentrer la lumière provenant de la zone éclairée de la surface de l'objet, un dispositif de détection de la lumière recevant la lumière recueillie et concentrée et une unité d'analyse permettant d'obtenir à partir de signaux provenant du dispositif de détection de la lumière un spectre Raman ou un spectre similaire,
**caractérisé en ce que**
la distance donnée est située dans la plage de 10 m à 100 m,
la substance dangereuse est constituée par des molécules d'un type défini, en particulier un explosif ou un composant d'un explosif,
le dispositif de détection de la lumière comporte une surface sensible à la lumière comprenant un ensemble de pixels individuels agencés sous la forme d'un réseau de pixels, chacun des pixels individuels fournissant des signaux de détection, et
l'unité d'analyse est susceptible de subdiviser la zone de détection en un ensemble de plus petites zones dites sous-zones, la dimension de chaque sous-zone étant comparable à la dimension d'une ou d'un petit nombre de particule(s) de molécules du type défini, de regrouper un ensemble de sous-zones pour former une plus grande zone, de détecter simultanément toutes les sous-zones individuelles comprises dans l'ensemble de l'image provenant du système optique représentée sur le dispositif de détection de la lumière et d'analyser individuellement les signaux de détection provenant de chacun des pixels individuels, chaque pixel correspondant à une sous-zone individuelle.

7. Système conforme à la revendication 6,
**caractérisé par**
un filtre permettant de filtrer la lumière recueillie avant sa réception par le dispositif de détection de la lumière de sorte que de la lumière d'une seule plage de longueurs d'onde soit détectée.

8. Système conforme à la revendication 7,
**caractérisé en ce que**
le filtre est réglable de sorte que le dispositif de détection de la lumière puisse détecter successivement la lumière pour chacune des plages de longueur d'onde d'un ensemble de plages de longueurs d'onde en réglant le filtre de façon correspondante.

9. Système conforme à l'une quelconque des revendications 6 à 8,
**caractérisé en ce que**
la source de lumière est agencée de sorte que la zone éclairée de la surface de l'objet soit une région linéaire ou une zone en forme de bande, en particulier une zone en forme de bande ayant une largeur correspondant à ou faiblement supérieure à la largeur ou à la hauteur d'une zone qui est représentée sur un seul pixel individuel.

10. Système conforme à la revendication 9,
**caractérisé par**
un dispositif de dispersion de longueurs d'onde en forme de bord ou linéaire agencé de sorte que la lumière recueillie et concentrée passe au travers, le dispositif de dispersion de longueurs d'onde étant un bord ou une ligne ayant un bord ou un axe orienté de sorte que la lumière recueillie provenant de la région linéaire frappe des lignes parallèles de pixels individuels et que la lumière recueillie provenant de la région linéaire frappant chacune de ces lignes renferme uniquement des longueurs d'onde d'une bande de longueurs d'onde individuelle correspondante.

11. Système conforme à la revendication 10,
**caractérisé en ce que**
le dispositif de dispersion de longueurs d'onde est une lentille à segments cylindriques, un prisme ou un réseau de dispersion.
